# EUROPÄISCHE PATENTANMELDUNG

(11) **EP 3 654 314 A1**
(43) Veröffentlichungstag der Anmeldung: **20.05.2020**
(21) Anmeldenummer: 19208293.1
(22) Anmeldetag: 11.11.2019
(51) Int. Cl.: G09B 5/06, G09B 19/00

(54) **VERFAHREN UND ELEKTRONISCHES SYSTEM ZUM UNTERSTÜTZEN EINER PERSON BEI DER AUSÜBUNG VON SKISPORT**

(30) Priorität: 13.11.2018 AT 509832018
(71) Anmelder: ATOMIC Austria GmbH, 5541 Altenmarkt im Pongau (AT)
(72) Erfinder: HOLZER, Helmut, 5600 St. Johann (AT); ROE, Jason, 5602 Wagrain (AT)
(74) Vertreter: Burger, Hannes

(57) **Zusammenfassung**

Die Erfindung betrifft ein Verfahren und ein elektronisches System zum Unterstützen einer Person (1) bei der Ausübung von Skisport. Das Verfahren umfasst eine Erfassung der derzeitigen sportlichen Leistungsfähigkeits- oder Geschicklichkeitswerte der sportausübenden Person durch ein elektronisches Erfassungssystem (3, 3'), welches elektronische Erfassungssystem (3, 3') ein von der Person (1) tragbares mobiles Endgerät (6), insbesondere ein Smartphone (7), umfasst. Weiters erfolgt eine Evaluierung der vom elektronischen Erfassungssystem (3, 3') bereitgestellten Zustandsinformationen oder Ist-Daten durch ein computerimplementiertes Evaluierungsmittel (21) und eine Bestimmung eines derzeitigen Niveaus an Leistungsfähigkeit oder Geschicklichkeit der Person (1). Daraufhin wird durch das computerimplementiere Evaluierungsmittel (21) eine Selektion oder Vorselektion von Lehrmaterial aus einer Vielzahl von für das computerimplementierte Evaluierungsmittel zugreifbaren Lehrmaterialien ausgeführt, wobei diese Selektion oder Vorselektion durch das computerimplementierte Evaluierungsmittel (21) unter Einbeziehung des automatisiert oder teilautomatisiert ermittelten Niveaus an Leistungsfähigkeit oder Geschicklichkeit der Person (1) vorgenommen wird. Voll- oder teilautomatisch selektiertes Lehrmaterial wird sodann unmittelbar auf dem von der sportausübenden Person (1) tragbaren mobilen Endgerät (6) ausgegeben.

## Beschreibung

Die Erfindung betrifft ein Verfahren zum Unterstützen einer Person, insbesondere eines Sportlers, bei der Ausübung von Skisport, wie dies in Anspruch 1 angegeben ist. Dieses Verfahren wird zumindest teilweise computerimplementiert ausgeführt, das heißt unter Einbindung von Computern bzw. softwaregesteuerten Recheneinheiten umgesetzt. Weiters betrifft die Erfindung ein elektronisches System zur Umsetzung des anspruchsgemäßen Verfahrens, wie es in Anspruch 13 angegeben ist.

Die AT517933B1, welche auf die Anmelderin zurückgeht, beschreibt einen Sportschuh für den Skilauf sowie ein hierbei eingesetztes elektronisches Kontrollsystem. Dieser Sportschuh umfasst einen zur Aufnahme des Fußes eines Benutzers vorgesehenen Innenschuh, eine den Innenschuh an dessen Außenseite zumindest abschnittsweise umgebende äußere Schale aus relativ hartem und formstabilen Kunststoff, und zumindest einen drucksensitiven Sensor zur Erfassung von mechanischen Drücken oder Kräften im oder am Sportschuh. Der Sportschuh weist weiters eine funktechnische Kommunikationsschnittstelle auf, welche zur drahtlosen Übertragung von Drucksignalen oder von dementsprechenden Daten des zumindest einen drucksensitiven Sensors ausgebildet ist. Diese funktechnische Kommunikationsschnittstelle am Sportschuh ist mit einer funktechnischen Kommunikationsschnittstelle an zumindest einer elektronischen Auswertevorrichtung kompatibel. Die elektronische Auswertevorrichtung ist zur Evaluierung der von dem zumindest einen drucksensitiven Sensor erfassten Drucksignale und der daraus abgeleiteten Druckverhältnisse im oder am Sportschuh vorgesehen. In Zusammenhang mit dem elektronischen Kontrollsystem soll dabei ein sogenannter digitaler Ski-Coach geschaffen sein, welcher Ski-Coach bzw. welches elektronische Kontrollsystem den Benutzer darin unterstützen soll, die Ausübung des entsprechenden Skilaufsportes zu optimieren bzw. komfortabler zu gestalten. Die mit den vorgestellten Maßnahmen erzielbaren Ergebnisse sind jedoch nur in Teilaspekten zufriedenstellend.

Die WO2007/015908A2 beschreibt ein System zur Anzeige der athletischen Laufleistung eines Laufsportlers auf elektronischen Geräten. Dabei werden die Signale eines Schrittsensors in der Sohle des Laufschuhs, eines Herzfrequenz- bzw. Blutdrucksensors am Körper des Läufers, und andere Sensordaten drahtlos an ein elektronisches Adaptergerät übertragen. Das Adaptergerät, welches zumindest die Schrittsignale empfängt, ist dabei über einen Stecker an die Datenschnittstelle eines standardmäßigen Anzeigegerätes mechanisch an- und abkoppelbar und übergibt via diese Datenschnittstelle die mittels dem Anzeigegerät zu visualisierenden Daten. Das Adaptergerät und das Anzeigegerät, welches durch ein standardmäßiges Mobiltelefon, einen PDA, einen MP3-player, eine Armbanduhr und dergleichen gebildet sein kann, werden am Körper des Läufers getragen und sind für Auswertungen der Laufleistung des Läufers vorgesehen. Dieses bekannte System ist in Zusammenhang mit der Ausübung von Skisport nur bedingt geeignet.

Die DE9417953U1 beschreibt eine Einlage für einen Skischuh. Diese Einlage soll als Lernhilfe für die Ausführung des richtigen Schwungs dienen. Sie besteht aus einem den Fersenbereich abdeckenden, flachen und flexiblen Einlegeteil. In diesem Einlegeteil ist eine mit einer Flüssigkeit gefüllte Zelle angeordnet, wobei in der Zelle ein auf Druck reagierender Sensor angeordnet ist, der über Kabel mit einer Stromversorgung und einem Controller außerhalb des Skischuhes verbunden ist. Der Controller wiederum ist mit einer Vorrichtung zum Erzeugen von akustischen Signalen, insbesondere einem Stereokopfhörer verbunden. Diese Vorrichtung ist als Unterstützung bei der Ausübung des Skisports nur bedingt zufriedenstellend.

Aufgabe der vorliegenden Erfindung war es, die Nachteile des Standes der Technik zu überwinden und Skiläufern eine technische Ausrüstung zur Verfügung zu stellen, die es ihnen ermöglicht, den Komfort bzw. die erzielbare Performance in Bezug auf die Sportausübung zu steigern.

Diese Aufgabe wird durch ein Verfahren und durch ein elektronisches System gemäß den Ansprüchen gelöst.

Das erfindungsgemäße, insbesondere computerimplementiert ausgeführte Verfahren zum Unterstützen einer sportausübenden Person, insbesondere eines Sportlers, in Zusammenhang mit der Ausübung von Skisport umfasst die Schritte:
- Erfassen der derzeitigen bzw. gegenwärtigen sportlichen Leistungsfähigkeits- oder Geschicklichkeitswerte der sportausübenden Person durch ein elektronisches Erfassungssystem, welches elektronische Erfassungssystem ein von der Person tragbares mobiles Endgerät, insbesondere ein Smartphone oder einen sonstigen Wearable-Computer, umfasst;
- Evaluieren der vom Erfassungssystem bereitgestellten Zustandsinformationen oder Ist-Daten durch ein computerimplementiertes Evaluierungsmittel;
- vollautomatisches Feststellen oder halbautomatisches Bestimmen eines derzeitigen Niveaus an Leistungsfähigkeit oder Geschicklichkeit der Person durch das computerimplementierte Evaluierungsmittel;
- Selektion oder Vorselektion von Lehrmaterial, beispielsweise von videobasiertem Trainingsmaterial, durch das computerimplementiere Evaluierungsmittel aus einer Vielzahl von für das computerimplementierte Evaluierungsmittel zugreifbaren oder bekannten, insbesondere verfügbaren Lehrmaterialien, wobei diese Selektion oder Vorselektion durch das computerimplementierte Evaluierungsmittel unter Berücksichtigung oder Einbeziehung des automatisiert oder teilautomatisiert ermittelten Niveaus an Leistungsfähigkeit oder Geschicklichkeit der Person vorgenommen wird;
- Ausgeben, insbesondere Anzeigen, Abspielen bzw. Wiedergeben von selektiertem Lehrmaterial, insbesondere in Form von Bildern und/oder Tonsignalen, unmittelbar auf dem von der sportausübenden Person tragbaren mobilen Endgerät.

Ein Vorteil der erfindungsgemäßen Maßnahmen liegt darin, dass eine hohe Qualität an Unterstützungsleistung für die sportausübende Person erreicht werden kann. Einerseits kann durch das elektronische Erfassungssystem ein fortlaufende bzw. hochaktuelle Erfassung der jeweiligen Leistungs- bzw. Geschicklichkeitswerte der jeweiligen, sportausübenden Person erreicht werden. Eine solche elektronische Ist-Zustand-Erfassung kann dabei auch vergleichsweise objektive und gegebenenfalls sogar tagesabhängig variierende Ergebnisse liefern. Insbesondere ist die Erfassung der Leistungs- bzw. Geschicklichkeitswerte der sportausübenden Person mithilfe der anspruchsgemäßen Maßnahmen nicht völlig von einer subjektiven Einschätzung bzw. Beurteilung der sportausübenden Person oder eines sonstigen Dritten abhängig. Hinzu kommt, dass durch das computerimplementierte Evaluierungsmittel ein möglichst optimales Trainings- bzw. Lehrmaterial - beispielsweise in Form von Video-Clips, Hörbüchern, kommentierten Bilderserien, Text- und/oder Sprachanweisungen - relativ zeitnah zur Verfügung steht und damit ein erhöhter Lern- bzw. Trainingseffekt erzielt werden kann. Das in Abhängigkeit der momentanen Leistungs- oder Geschicklichkeitswerte der Person jeweils selektierte oder vorselektierte Trainings- bzw. Lehrmaterial kann dabei entweder vollautomatisch, oder mit möglichst geringer Benutzerinteraktion, also teilautomatisch bereitgestellt werden, wodurch für die sportausübende Person Zeiteinsparungen und zugleich Komfortsteigerungen erzielt werden können. Außerdem kann der sportausübenden Person bzw. dem Benutzer des angegebenen Systems durch die zumindest teilautomatisiert ausgewählten Trainings- bzw. Lehrmaterialien eine möglichst maßgeschneiderte Unterstützung geboten werden. Darüber hinaus kann die jeweilige Hilfestellung an nahezu beliebigen Orten und zu jeweils geeignet erscheinenden Zeitpunkten genutzt werden, wodurch der Lern- bzw. Trainingserfolg für die sportausübende Person nochmals gesteigert werden kann.

Insbesondere kann es zweckmäßig sein, wenn eine automatisierte Selektion oder Vorselektion von beispielsweise videobasiertem Lehrmaterial vorgesehen ist, welches Lehrmaterial zur Verbesserung der derzeitigen sportlichen Leistungsfähigkeits- oder Geschicklichkeitswerte der sportausübenden Person, das heißt des Benutzers des Systems, beitragen kann. Damit kann das jeweilige Lehrmaterial zielgerichtet, möglichst fehlerfrei und auch rasch ermittelt werden und sodann der sportausübenden Person zur Verfügung gestellt werden.

Entsprechend einer vorteilhaften Maßnahme kann eine Anzeige von Videobildern und/oder textbasierten Kommentaren auf einem Display des tragbaren mobilen Endgerätes vorgesehen sein. Alternativ oder in Kombination dazu kann eine Ausgabe von Sprachinstruktionen mittels einem Lautsprecher des tragbaren mobilen Endgeräts vorgesehen sein. Dadurch können die Kosten zur Schaffung des angegebenen technischen Systems möglichst gering gehalten werden. Insbesondere ist die Entwicklung einer proprietären elektronischen Ausgabe-Hardware nicht erforderlich, wodurch eine hohe Nutzungsakzeptanz erreicht werden kann bzw. eine kurze Einlern- bzw. Gewöhnungsphase bei Nutzern des angegebenen Systems erzielt werden kann.

Darüber hinaus kann vorgesehen sein, dass videobasiertes Lehrmaterial via eine erste Daten- oder Kommunikationsschnittstelle des tragbaren mobilen Endgeräts abgerufen wird, welche Daten- oder Kommunikationsschnittstelle zur Fernübertragung und zum Fernempfang von Daten gegenüber zumindest einem Hostrechner oder Internetportal ausgebildet ist. Die Datenschnittstelle kann dabei nach dem WLAN-Standard oder gemäß einem ausreichend leistungsfähigen Mobilfunknetz-Standard ausgeführt sein. Dadurch kann das Angebot bzw. Volumen an Lehrmaterial ständig erweitert bzw. angepasst werden, ohne dass periodische oder bedarfsabhängige Aktualisierungen am mobilen Endgerät erforderlich wären. Zudem kann dadurch aus einem hohen Volumen an Lehrmaterial ausgewählt werden, ohne dass lokale Speicherressourcen des mobilen Endgeräts belastet werden.

Vorteilhaft ist auch eine Ausprägung, gemäß welcher Grafiken, Symbole oder Hinweistexte einem videobasierten Lehrmaterial darstellerisch überlagert werden, wobei diese Überlagerung in Abhängigkeit von automatisiert festgestellten Ausführungsdefiziten der zu unterstützenden Person vorgenommen wird. Dadurch kann eine zielgerichtete und spezifische Unterstützung des Sportlers zur Vermeidung von bestimmten bzw. wiederholt vorgekommenen Ausführungsfehlern erreicht werden.

Gemäß einer Weiterbildung kann eine computerimplementierte Ermittlung und Ausgabe einer Ermüdungsquantifikation und/oder Pausenempfehlung am tragbaren mobilen Endgerät vorgesehen sein. Dadurch kann die persönliche Sicherheit der sportausübenden Person selbst, aber auch von Personen, welche sich in der näheren Umgebung befindlichen, verbessert werden. Insbesondere können allmähliche Ermüdungserscheinungen durch das elektronische Erfassungssystem möglichst objektiv bzw. quantitativ ermittelt werden und kann die sportausübende Person bzw. ein sonstiger Benutzer des Systems, beispielsweise ein Trainer, auf allmähliche Ermüdungen und damit einhergehende Risiken hingewiesen werden. Außerdem können dadurch Trendanalysen und Leistungsfähigkeitsentwicklungen vorgenommen werden.

Ferner kann es zweckmäßig sein, wenn eine Ausgabe von visuell, akustisch und/oder taktil wahrnehmbaren Signalen mittels dem Display, dem Lautsprecher und/oder dem Vibrationsgenerator des tragbaren mobilen Endgeräts vorgenommen wird. Dadurch kann die jeweils am geeignetsten erscheinende Sinneswahrnehmung der sportausübenden Person genutzt werden, um in Interaktion treten zu können bzw. um der sportausübenden Person relevante Informationen bzw. Zustände zuverlässig mitteilen zu können. Zudem können die Hardwarekosten zur Umsetzung des angegebenen Systems bzw. Verfahrens durch Nutzung von standardmäßigen bzw. handelsüblichen, programmierbaren Endgeräten möglichst gering gehalten werden.

Darüber hinaus kann vorgesehen sein, dass Parameter ausgewählt aus der Gruppe umfassend Ausübungszeitdauer, Geschwindigkeitsprofil, Beschleunigungsprofil, Kräfteprofil, Sportgeräte-Aufkantwinkel, Skistil, Wetterverhältnisse wie beispielsweise kalt, warm, Nebel, Schneefall und dergleichen, Pistenzustände wie beispielsweise Tiefschnee, hart, weich, eisig, griffig, und Pistenschwierigkeitsgrade, wie beispielsweise blaue, rote oder schwarze Kategorie und Off-Piste, für die computerimplementierte Ermittlung einer Ermüdungsquantifikation und/oder Pausenempfehlung einbezogen werden.

Des Weiteren kann eine Hinterlegung von erfassten Leistungs- oder Geschicklichkeitswerten und/oder von festgestellten Niveaus an Leistungsfähigkeit oder Geschicklichkeit in einer lokalen Speichervorrichtung des tragbaren mobilen Endgeräts und/oder in einer externen, fernzugreifbaren Speichervorrichtung vorgesehen sein. Dadurch können die sport- bzw. ausübungsspezifischen Daten zuverlässig gesammelt und für spätere Auswertungen und Rückschau-Aufgaben bereitgehalten werden.

Gemäß einer weiteren Ausprägung können zeitlich zurückliegende, in einer Speichervorrichtung hinterlegte Leistungs- oder Geschicklichkeitswerte und/oder festgestellte Niveaus an Leistungsfähigkeit oder Geschicklichkeit mit derzeitigen Leistungs- oder Geschicklichkeitswerten und/oder Niveaus an Leistungsfähigkeit oder Geschicklichkeit mittels dem computerimplementierten Evaluierungsmittel verglichen werden. Insbesondere sind dadurch relativ detailreiche Trendanalysen ermöglicht, mit welchen die jeweilige Entwicklung der sportausübenden Person gut evaluiert bzw. nachvollzogen werden kann.

Ferner kann ein Vergleichen von Leistungs- oder Geschicklichkeitswerten und/oder von festgestellten Niveaus an Leistungsfähigkeit oder Geschicklichkeit in Bezug auf die einer vordefinierten Vergleichsgruppe angehörenden Personen vorgesehen sein. Ein solcher Vergleich kann beispielsweise mit einem Profi-Sportler, beispielsweise einem Weltcup-Teilnehmer, oder mit einer x-beliebigen Person aus einer Community vorgenommen werden. Insbesondere kann dadurch ein Competition-Effekt bzw. ein Wettbewerbs-Anreiz geschaffen werden, wodurch die sportliche Leistungsfähigkeit und/oder Motivation von sportausübenden Personen gegebenenfalls weiter gesteigert werden kann.

Das elektronische Erfassungssystem kann entsprechend einer vorteilhaften Ausführungsform wenigstens einen Druckerfassungssensor umfassen, welcher in oder an einem von der Person tragbaren Sportschuh angeordnet ist. Dabei kann wenigstens eine elektronische Auswertungseinheit vorgesehen sein, welche die elektrischen Drucksignale des wenigstens einen Druckerfassungssensors auswertet oder eine Vorverarbeitung bzw. Konditionierung der elektrischen Drucksignale ausführt. Damit sind tiefgreifende Rückschlüsse auf das Bewegungsverhalten der sportausübenden Person erzielbar, ohne dass eine komplexe Kompensation eines Driftverhaltens des zumindest einen Druckerfassungssensors erforderlich wäre. Außerdem kann ein entsprechendes Druckerfassungssystem im bzw. am Sportschuh relativ robust und langzeitstabil umgesetzt werden.

Die elektronische Auswertungseinheit kann ein Bestandteil bzw. eine Teilkomponente des elektronischen Erfassungssystems sein. Die elektronische Auswertungseinheit kann dazu eingerichtet sein, vorzugsweise an zumindest einem Sportschuh der sportausübenden Person befestigt zu werden, oder an einer sonstigen Stelle von der sportausübenden Person getragen zu werden.

Die Aufgabe der Erfindung wird weiters durch ein elektronisches System gelöst, wie es in den Ansprüchen angegeben ist. Die damit erzielbaren technischen Wirkungen und vorteilhaften Effekte sind den vorhergehenden und den nachstehenden Beschreibungsteilen zu entnehmen.

Zum besseren Verständnis der Erfindung wird diese anhand der nachfolgenden Figuren näher erläutert.

Es zeigen jeweils in stark vereinfachter, schematischer Darstellung:
- Fig. 1: ein Ausführungsbeispiel eines elektronischen Systems zur Unterstützung einer Person bei der Ausübung von Skisport;
- Fig. 2: ein Blockschaltbild eines Ausführungsbeispiels eines elektronischen, computerimplementierten Systems zur Auswahl oder Vorauswahl von optimiertem oder geeignetem Lehrmaterial für eine sportausübende Person;
- Fig. 3: ein Blockschaltbild eines Ausführungsbeispiels eines elektronischen, computerimplementierten Systems zur Unterstützung einer Person bei der Ausübung von Skisport.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

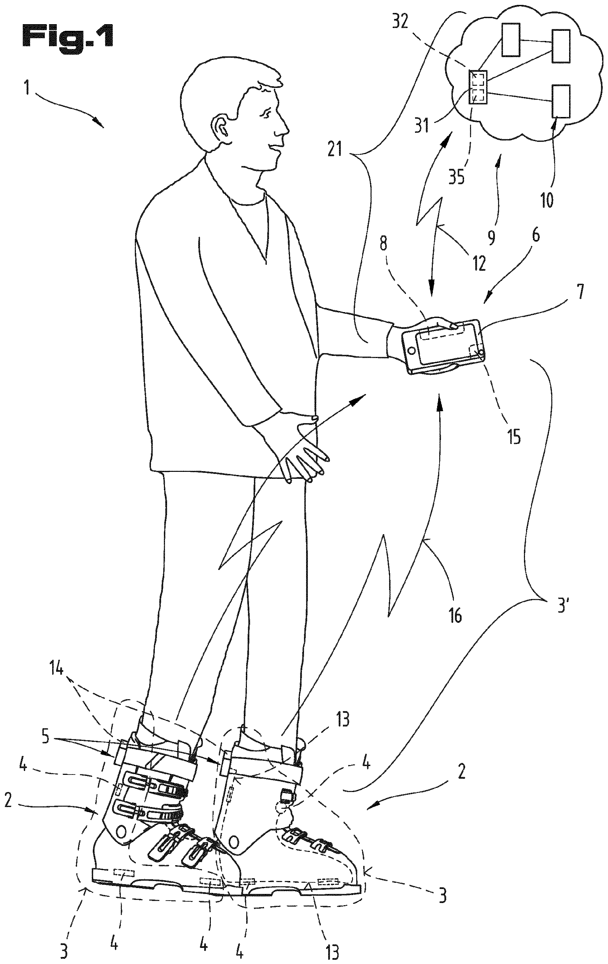

Fig. 1 zeigt eine Person 1, welche technische Ausrüstungsgegenstände zur Ausübung von Skisport trägt bzw. nutzt. Anstelle der beispielhaft dargestellten Sportschuh-Ausrüstung zur Ausübung von Alpin-Skisport, kann das nachfolgend vorgestellte System auch in Zusammenhang mit einer Ausübung von Langlauf-Skisport, Snowboard-Skisport oder Tourenlauf-Skisport angewandt bzw. eingesetzt werden.

Die in Fig. 1 dargestellte Person 1, welche beispielsgemäß für die Ausübung von Alpin-Skisport ausgerüstet ist, wird durch das nachfolgend beschriebene System und Verfahren in der entsprechenden Sportausübung unterstützt bzw. durch die beschriebenen technischen Mittel gecoacht bzw. trainiert. Die Person 1 trägt dabei ein Paar Sportschuhe 2, beispielsgemäß Alpin-Skischuhe, welche mit einem nicht dargestellten Paar von Skiern, beispielsgemäß Alpin-Skiern (nicht dargestellt), bedarfsweise koppelbar und davon entkoppelbar sind.

Zumindest einer der Sportschuhe 2, vorzugsweise jeder Sportschuh des Sportschuh-Paares, weist zumindest Teilkomponenten eines elektronischen Erfassungssystems 3, 3' auf. Zweckmäßig ist es, wenn das elektronische Erfassungssystem 3, 3' wenigstens einen Druckerfassungssensor 4 in und/oder an dem wenigstens einen, von der Person tragbaren Sportschuh 2, wenigstens eine elektronische Vorerarbeitungs- bzw. Auswertungseinheit 5 für die Drucksingale des wenigstens einen Druckerfassungssensors 4, und ein von der Person 1 tragbares, mobiles Endgerät 6 umfasst. Zweckmäßig ist es, wenn das mobile Endgerät 6 durch ein standardmäßiges bzw. handelsübliches Smartphone (Mobiltelefon) 7 gebildet ist. Dies vor allem dann, wenn das mobile Endgerät 6 der sportausübenden Person 1 zugewiesen ist bzw. von dieser während der Sportausübung zu tragen ist. Das mobile Endgerät 6 kann aber auch durch einen Tablet-Computer gebildet sein bzw. einen solchen umfassen. Dies vor allem dann, wenn eine vergleichsweise größere Anzeigefläche zweckmäßig ist. Ein solches mobiles Endgerät 6 kann zweckmäßigerweise einem Trainer zugewiesen sein bzw. einem die sportausübende Person 1 zusätzlich unterstützenden Coach zugeordnet sein. Vor allem das tragbare mobile Endgerät 6, welches der sportausübenden Person 1 zugeordnete ist, soll durch einen Wearable-Computer definiert sein, beispielsweise durch ein Smartphone 7, durch eine Smartwatch und/oder durch Smartglasses (Datenbrille) gebildet sein.

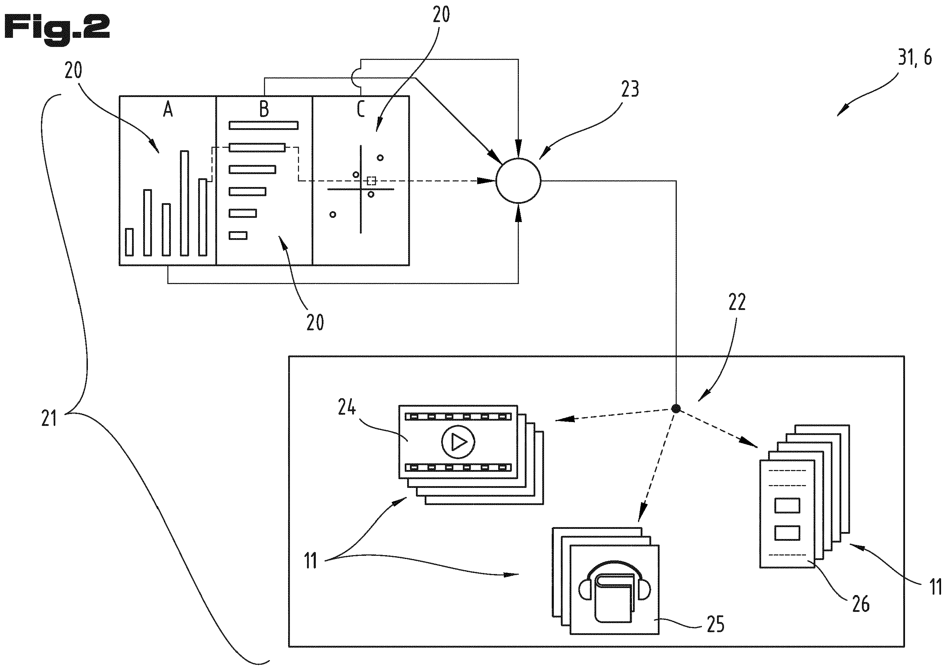

Das jeweilige mobile Endgerät 6 weist wenigstens eine erste Kommunikationsschnittstelle 8 zur datentechnischen Kommunikation mit dem Internet 9 bzw. mit einem weitläufig entfernt liegenden Rechnersystem 10 auf. Diese erste datentechnische Kommunikationsschnittstelle 8 ist dabei zur bidirektionalen Datenübertragung ausgebildet, insbesondere zum Herunterladen von umfangreichen Informationen bzw. umfassenden Lehrmaterialien 11 - Fig. 2 - vorgesehen. Die erste Daten- bzw. Kommunikationsschnittstelle 8 ist somit bevorzugt zum Aufbau einer Internet-Datenverbindung 12 eingerichtet und kann durch eine standardmäßige Daten-Kommunikationsschnittstelle 8 eines Smartphones 7 definiert sein. Demnach ist hier eine drahtlose Datenübertragungsstrecke bzw. Kommunikationsschnittstelle 8 vorgesehen.

Die wenigstens eine elektronische Auswerteeinheit 5 für die von dem wenigstens einen Drucksensor 4 bereitgestellten Druck- bzw. Kraftsignale kann, wie schematisch dargestellt, schuhseitig positioniert sein, oder an einer anderen Position von der Person 1 getragen werden. Die elektronische Auswerteeinheit 5 kann aber auch entfallen. Dies insbesondere dann, wenn die komplette Signalverarbeitung von dem mobilen elektronischen Erfassungssystem 3', insbesondere von dem Smartphone 7 der Person 1 und von dem zumindest einen Druckerfassungssensor 4 unmittelbar vorgenommen wird. In diesem Fall weisen die Druckerfassungssensoren 4 im bzw. am Sportschuh 2 jeweils funktechnische Kommunikationsschnittstellen zur Daten- bzw. Signalübertragung zum mobilen Endgerät 6 auf. Insbesondere sind in diesem Fall die Druckerfassungssensoren 4 dazu eingerichtet, die jeweiligen Druck- bzw. Kraftwerte unmittelbar an eine drahtlose Nahbereichs-Kommunikationsschnittstelle 15 des mobilen Endgeräts 6 bzw. des Smartphones 7 zu übertragen.

Entsprechend einer zweckmäßigen Ausführungsform ist jedoch vorgesehen, dass die Druckerfassungssensoren 4 am bzw. im Sportschuh 2 die jeweiligen elektrischen Signale via elektrische Leitungsverbindungen 13 an die schuhseitig bzw. personenseitig positionierte, elektronische Vorverarbeitungs- bzw. Auswertungseinheit 5 übertragen. Vorzugsweise findet dann in der elektronischen Auswertungseinheit 5 zumindest eine signaltechnische Vorverarbeitung bzw. eine Komprimierung und datentechnische Aufbereitung der Drucksignale bzw. Druckwerte, welche von der Mehrzahl an Druckerfassungssensoren 4 detektiert werden, statt. Zweckmäßig ist es, wenn zumindest zwei, vorzugsweise vier oder mehr über die Flächenbereiche des Sportschuhs 2 verteilt angeordnete Druckerfassungssensoren vorgesehen sind.

Die bevorzugt paarweise, also für jeden Sportschuh 2 vorgesehene, elektronische Auswertungseinheit 5 umfasst jeweils zumindest eine funktechnische Nahbereichs-Kommunikationsschnittstelle 14. Diese Nahbereichs-Kommunikationsschnittstellen 14 sind zur daten- bzw. signaltechnischen Kommunikation mit einer korrespondierenden, datentechnischen Nahbereichs-Kommunikationsschnittstelle 15 am mobilen Endgerät 6 vorgesehen. Diese Nahbereichs-Kommunikationsschnittstellen 14, 15 können beispielsweise durch Kommunikationsschnittstellen nach dem WLAN-, RFID-, Bluetooth-, oder ZigBee-Standard ausgeführt sein, oder gemäß einer sonstigen standardisierten, datentechnischen Kommunikationstechnologie umgesetzt sein. Via diese korrespondierenden Nahbereichs-Kommunikationsschnittstellen 14, 15 können die Signalwerte bzw. Daten der Druckerfassungssensoren 4 über zumindest eine schematisch dargestellte Nahbereichs-Kommunikationsstrecke 16 drahtlos an das mobile Endgerät 6 übertragen werden.

Zumindest anhand dieser Drucksignale von den Druckerfassungssensoren 4 können auswertungstechnische Rückschlüsse auf das Nutzungsverhalten bzw. auf die Performance in der Sportausübung der Person 1 Rückschluss gezogen werden. Insbesondere dann, wenn die Druckerfassungssensoren 4 mehrfach ausgeführt sind und an verteilt angeordneten Positionen der Sportschuhe 2 angeordnet sind, wie dies seitens der Anmelderin aus der AT517933B1 bekannt ist. Insbesondere kann mit den Druckerfassungssensoren 4 im Zuge der Ausübung von Skisport detektiert werden, welche Gewichtsverteilung, Dynamik, Impulsivität und Kräfteverteilung zwischen der sportausübenden Person 1 und dem jeweiligen Sportgerät, insbesondere einem Paar von Skiern, und/oder dem jeweiligen Untergrund jeweils ausgeübt wird bzw. in zeitlicher Hinsicht jeweils vorliegt.

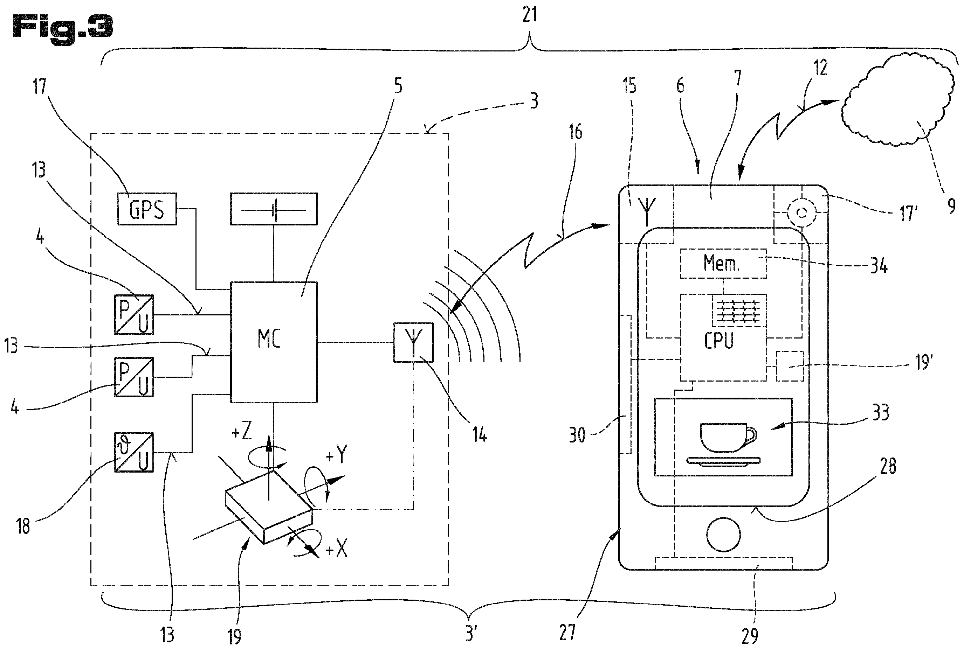

Neben drucksensitiven Sensoren 4 können eine Mehrzahl weiterer Sensoren zur datentechnischen Erfassung des Nutzungsverhaltens bzw. der sportlichen Leistungsfähigkeit der Person 1 am Sportschuh 2 und/oder am jeweiligen Sportgerät und/oder unmittelbar an der Person 1 ausgebildet bzw. angeordnet sein. Wie auch am besten aus Fig. 3 ersichtlich ist, kann das elektronische Erfassungssystem 3,3' auch Sensoren bzw. Erfassungssysteme ausgewählt aus folgender Gruppe umfassen: Beschleunigungssensoren, lokale und/oder globale Positionserfassungssysteme 17, 17', Temperatursensoren 18, gyroskopische Sensoren 19, kontaktdetektierende Sensoren bzw. Schalter, Geschwindigkeitssensoren und Zeitgeber. Durch Auswertung und Korrelierung der jeweiligen Sensorsignale bzw. Erfassungssysteme kann ein umfassendes Abbild über das Nutzungsverhalten bzw. über momentane sportliche Leistungsfähigkeits- oder Geschicklichkeitswerte 20 der jeweiligen, sportausübenden Person 1 gewonnen werden (Fig. 2).

Insbesondere kann vorgesehen sein, dass das elektronische Erfassungssystem 3' auf Sensoriken zurückgreift bzw. Sensorsysteme einbezieht, welche standardmäßig im mobilen Endgerät 6, insbesondere im Smartphone 7 implementiert sind und ausgewählt sind aus der Gruppe umfassend lokale und/oder globale Positionserfassungssysteme 17', gyroskopische Sensoren 19', inertiale Messeinheiten (IMU), Beschleunigungssensoren und Neigungssensoren, wie dies in Fig. 3 schematisch ersichtlich ist. Dadurch bestehen umfassende Erfassungs- bzw. Detektionsmöglichkeiten, welche auf erprobten Sensoriken basieren, eine hohe funktionale Zuverlässigkeit besitzen und darüber hinaus relativ kostengünstig genutzt bzw. mitbenutzt werden können.

Die Leistungsfähigkeits- oder Geschicklichkeitswerte 20 können dabei Parameter ausgewählt aus einer Gruppe umfassend Gleichgewichtsverhalten (Balance), Aufkantwinkel der Skier, Dynamik der Richtungsänderungen, Geschwindigkeitsprofil, Kräfteverhältnisse zwischen Person 1 und Sportschuh 2, Gewichts- bzw. Lastverteilung zwischen linkem und rechtem Sportschuh 2, und dergleichen, umfassen. Die vom elektronischen Erfassungssystem 3, 3' bereitgestellten bzw. detektierten, gegenwärtigen Leistungsfähigkeits- oder Geschicklichkeitswerte 20, insbesondere die derzeitigen Zustandsinformationen oder Ist-Daten über die sportausübende Person 1, werden sodann von einem computerimplementierten Evaluierungsmittel 21 bewertet bzw. evaluiert. Das computerimplementierte Evaluierungsmittel 21 kann durch eine einzelne Rechnereinheit oder durch einen Verbund bzw. eine Mehrzahl von entsprechend programmierten, miteinander kommunizierenden Rechnereinheiten definiert sein. Zweckmäßig ist es, wenn dieses computerimplementierte Evaluierungsmittel 21 einerseits das mobile Endgerät 6 und andererseits ein peripheres Rechnersystem 10, insbesondere einen dezidierten Server für das vorgestellte Evaluierungssystem umfasst (Fig. 1). Hierfür sind am mobilen Endgerät 6, insbesondere am Smartphone 7, programmtechnische Mittel (softwaretechnische Applikationen) implementiert und ausführbar. Analog dazu sind am dem vorzugsweise via Internet 9 zugreifbaren bzw. kommunizierenden Rechnersystem 10, insbesondere am dezidierten Server des Systembetreibers, entsprechende programmtechnische Mittel, insbesondere softwaretechnisch umgesetzte Betriebs- und Evaluierungsprogramme, implementiert und ausführbar.

Mittels dem softwaretechnisch bzw. programmgesteuert betreibbaren, computerimplementierten Evaluierungsmittel 21 - Fig. 1--3 - ist eine vollautomatische Feststellung oder zumindest eine halbautomatische Bestimmung eines derzeitigen Niveaus an Leistungsfähigkeit oder Geschicklichkeit des Systemnutzers bzw. der sportausübenden, mit dem elektronischen Erfassungssystem 3, 3' ausgestatteten Person 1 ermöglicht.

Das computerimplementierte Evaluierungsmittel 21 ist weiters dazu eingerichtet, eine Selektion oder Vorselektion von geeignetem Lehrmaterial 11 für die jeweilige, sportausübende Person 1, insbesondere von beispielsweise videobasiertem Trainingsmaterial, vorzunehmen. Hierfür wird - wie in Fig. 2 veranschaulicht - ein voll- oder teilautomatischer Selektions- oder Vorselektionsschritt 22 ausgeführt. Dieser kann durch standardisierte, aus dem Stand der Technik bekannte Prozeduren, insbesondere durch Wertungs- und Auswahlprozeduren umgesetzt sein. In diese Wertungs- bzw. Auswertungsvorgänge fließen dabei insbesondere die jeweils ermittelten, möglichst aktuellen Leistungsfähigkeits- oder Geschicklichkeitswerte 20 der Person 1 ein. Hierzu wird - wie in Fig. 2 veranschaulicht - ein programmtechnisch implementierter Gewichtungs- und/oder Zusammenführungsschritt 23 ausgeführt. In diesem Gewichtungs- und/oder Zusammenführungsschritt 23 werden die jeweiligen Geschicklichkeits- und/oder Leistungsfähigkeitsdaten der Person 1 programmtechnisch verarbeitet bzw. verwertet. Der voll- oder teilautomatische Selektionsschritt und/oder der voll- oder teilautomatische Vorselektionsschritt 22, welcher mittels dem computerimplementierten Evaluierungsmittel 21 zur Gänze bzw. zumindest teilweise ausführbar ist, erfolgt dabei unter Berücksichtigung oder Einbeziehung des automatisiert oder teilautomatisiert ermittelten Niveaus an Leistungsfähigkeit oder Geschicklichkeit der Person 1, insbesondere unter automatisierter Berücksichtigung und Evaluierung der momentanen bzw. letztgültigen Leistungsfähigkeits- oder Geschicklichkeitswerte 20 der jeweiligen Person 1.

Der entsprechende Auswahl- oder Vorauswahlprozess wird dabei innerhalb eines definierten, relativ umfangreich vorhandenen Lehrmaterial-Volumens 11 vorgenommen. Der Selektionsschritt 22 kann dabei vollautomatisch durch das computerimplementierte Evaluierungsmittel 21 und/oder durch das elektronisch Erfassungssystem 3, 3'umgesetzt sein, das heißt ohne nennenswerte Interaktion von Seiten der sportausübenden Person 1 oder von Seiten einer sonstigen Person vorgenommen werden. Alternativ oder in Kombination dazu ist ein Vorselektionsschritt 22 denkbar, welcher unter Einbeziehung von Eingaben der sportausübenden Person 1 oder eines sonstigen Dritten letztendlich zu einer Selektion von geeignetem bzw. als adäquat erachteten Lehrmaterial 11 führt.

Das insgesamt für eine Auswahl zur Verfügung stehende Lehrmaterial 11 kann dabei Videos 24 (videobasiertes Lehrmaterial 11) und/oder Hörbücher 25 (tonbasiertes Lehrmaterial 11) und/oder Informationsdokumente 26 (text- und/oder bildbasiertes Lehrmaterial 11) umfassen. Das bzw. die vom Evaluierungsmittel 21 in Anbetracht der jeweiligen Leistungs- oder Geschicklichkeitswerte 20 am geeignetsten bzw. am hilfreichsten angesehene, letztendlich ausgewählte Lehrmaterial 11 wird sodann der sportausübenden Person 1 und/oder einem sonstigen Dritten empfohlen und kann sogleich oder zu einem späteren Zeitpunkt auf dem von der Person 1 tragbaren mobilen Endgerät 6 ausgegeben werden. Diese Ausgabe kann steuerungstechnisch initiiert und letztendlich von der Person 1 freigegeben bzw. aktiviert werden. Alternativ oder in Kombination dazu kann vorgesehen sein, dass die entsprechende Ausgabe vollautomatisch startet, also ohne vorheriger Benutzerinteraktion ausgeführt wird. Insbesondere kann vorgesehen sein, dass nach einer entsprechenden Empfehlung von Seiten des Evaluierungsmittels 21, welche vorzugsweise via das mobile Endgerät 6 ausgegeben wird, ein ausdrücklicher Startbefehl zur Ausgabe des entsprechenden Lehrmaterials 11 in das mobile Endgerät 6 einzugeben ist.

Typischerweise sind dutzende, hunderte oder sogar tausende Informations- bzw. Datenobjekte in Zusammenhang mit facheinschlägigen bzw. disziplinspezifischen Lehrmaterialien 11 verfügbar. Das computerimplementierte Evaluierungsmittel 21 ist dabei dazu eingerichtet, aus einer hohen Anzahl von grundsätzlich verfügbaren Lehrmaterialien 11 eine geeignete Auswahl oder Vorauswahl zu treffen und damit der sportausübenden Person 1 relativ rasch und zielgerichtet eine Hilfestellung zu bieten. Von besonderem Nutzen ist dabei auch, dass diese Hilfestellung in Form von möglichst adäquatem Lehrmaterial 11 relativ zeitnah erfolgen kann, insbesondere unmittelbar nach einer Sportausführung bzw. nach einer kurzen Sportsequenz bereitgestellt wird, oder auch während der Sportausübung bereitgestellt werden kann. Die Verfügbarkeit des jeweiligen, möglichst optimal unterstützenden Trainings- bzw. Lehrmaterials 11 ist dabei nahezu unabhängig vom jeweiligen Aufenthaltsort der sportausübenden Person 1, sofern ausreichend stabile und leistungsfähige datentechnische Kommunikationsverbindungen vorliegen.

Die Ausgabe des jeweiligen Lehrmaterials 11 erfolgt dabei auf einer standardmäßig implementierten Ausgabevorrichtung 27 des mobilen Endgeräts 6. Eine solche Ausgabevorrichtung 27 kann ein Display 28, einen Lautsprecher 29 und/oder einen Vibrationsgenerator 30 umfassen (Fig. 3).

Insbesondere ist das mobile Endgerät 6 dazu eingerichtet, Videobilder und/oder textbasierte Kommentare, insbesondere Textanweisungen, auf dessen Display 28 anzuzeigen und weiters eine Ausgabe von Tonsignalen und/oder und Sprachinstruktionen mittels dem Lautsprecher 29 vorzunehmen. Dadurch kann der Informationsgehalt bzw. der Lernerfolg und Lernfortschritt für jene Person 1, welche das angegebene elektronische System nutzt, deutlich gesteigert werden.

Die voll- oder teilautomatisierte Selektion oder Vorselektion von Lehrmaterial 11 mittels dem computerimplementierten Evaluierungsmittel 21 erfolgt dabei unter dem Gesichtspunkt der Verbesserung und/oder Stabilisierung der derzeitigen, sportlichen Leistungsfähigkeits- oder Geschicklichkeitswerte 20 des Benutzers des angegebenen Systems, insbesondere der sportausübenden Person 1.

Weiters sind am mobilen Endgerät 6 programmtechnische Mittel ausführbar, welche ein Abrufen von Lehrmaterial 11 (Download) via die Daten- bzw. Kommunikationsschnittstelle 8 des tragbaren, mobilen Endgeräts 6 ermöglichen. Insbesondere ist diese Daten- bzw. Kommunikationsschnittstelle 8 zur Fernübertragung und zum Fernempfang von Daten gegenüber zumindest einem systemzugehörigen Hostrechner 31 oder einem entsprechenden Internetportal 32 - Fig. 1 - ausgebildet. Insbesondere kann auf einen systemzugehörigen Hostrechner 31 via die vorstehend beschriebene Internet-Datenverbindung 12 Zugriff erlangt werden. Die Daten- bzw. Kommunikationsschnittstelle 8 des mobilen Endgeräts 6 kann dabei durch eine Schnittstelle nach dem WLAN-Standard und/oder durch eine Schnittstelle nach einem Mobilfunknetz-Standard mit ausreichender Datenübertragungsrate gebildet sein.

Entsprechend einer zweckmäßigen Maßnahme kann das mobile Endgerät 6 dazu eingerichtet sein, eine darstellerische Überlagerung von Grafiken, Symbolen oder Hinweistexten gegenüber auszugebendem Lehrmaterial 11, insbesondere in Bezug auf Bild- oder Videomaterial 24, vorzunehmen. Diese darstellerische Überlagerung ist dazu vorgesehen, in Abhängigkeit von automatisiert festgestellten Ausführungsdefiziten der zu unterstützenden, sportausübenden Person 1 eine zielgerichtete Hilfestellung zur sportlichen Verbesserung der Person 1 zu erzielen.

Weiters kann das mobile Endgerät 6 bzw. ein darauf ausführbares programmtechnisches Mittel dazu eingerichtet sein, eine computerimplementierte Ermittlung und Ausgabe einer Pausempfehlung 33 vorzunehmen. Diese Pausenempfehlung 33 kann dabei durch Aktivierung von zumindest einer der Ausgabevorrichtungen 27 erfolgen, beispielsweise durch Ansteuerung des Displays 28 und/oder des Lautsprechers 29 und/oder des Vibrationsgenerators 30 abgesetzt werden. Alternativ oder in Kombination zur Ermittlung und Ausgabe einer Pausenempfehlung 33 kann auch die Erfassung und Ausgabe einer Ermüdungsquantifikation mittels dem tragbaren mobilen Endgerät 6 vorgesehen sein. Die Ergebnisse der Pausenempfehlung 33 und/oder der Ermüdungsquantifikation basieren dabei auf den vom elektronischen Erfassungssystem 3, 3' erfassten Werten und/oder auf den vom computerimplementierten Erfassungsmittel 21 gewonnen Erkenntnissen bzw. aufgezeichneten Parametern über die sportausübende Person 1.

Insbesondere können Interaktionen zwischen der Person 1 und dem vorgestellten, technischen System durch Ausgabe von visuell, akustisch und/oder taktil wahrnehmbaren Signalen eingeleitet werden, das heißt mittels dem Display 28, dem Lautsprecher 29 und/oder dem Vibrationsgenerator 30 des tragbaren, mobilen Endgeräts 6 vorgenommen werden. In eine computerimplementierte Ermittlung einer Ermüdungsquantifikation und/oder Pausenempfehlung 33 für die sportausübende Person 1 können dabei folgende Parameter ausgewählt aus der Gruppe umfassend Ausübungszeitdauer, Geschwindigkeitsprofil, Beschleunigungsprofil, Kräfteprofil, Sportgeräte-Aufkantwinkel, Wetterverhältnisse wie zum Beispiel kalt, warm, Nebel, Schneefall und dergleichen, Pistenzustände wie zum Beispiel ebenflächig, buckelig, Tiefschnee, harter, weicher, eisiger, griffiger Schnee und dergleichen, einbezogen werden.

Das angegebene elektronische System kann auch dazu eingerichtet sein, eine Hinterlegung von erfassten Leistungs- oder Geschicklichkeitswerten 20 bzw. von festgestellten Niveaus an Leistungsfähigkeit oder Geschicklichkeit in einer lokalen Speichervorrichtung 34 - Fig. 3 - des tragbaren, mobilen Endgeräts 6 vorzunehmen und/oder in einer externen, fernzugreifbaren Speichervorrichtung 35 - Fig. 1 - vorzunehmen.

Das computerimplementierte Evaluierungsmittel 21 und/oder das elektronische Erfassungssystem 3, 3', insbesondere in Verbindung mit darauf ausführbaren programmtechnischen bzw. softwarebasierten Mitteln (hinterlegter Programmcode), kann auch zum Vergleichen von zeitlich zurückliegenden, in der Speichervorrichtung 34 und/oder 35 hinterlegten Leistungsfähigkeits- oder Geschicklichkeitswerten 20 und/oder von historisch festgestellten Niveaus an Leistungsfähigkeit oder Geschicklichkeit mit derzeitigen Leistungsfähigkeits- oder Geschicklichkeitswerten und/oder Niveaus an Leistungsfähigkeit oder Geschicklichkeit eingerichtet sein. Dadurch lassen sich Trendanalysen erzeugen und sportliche Verbesserungen bzw. Stabilisierungen im Hinblick auf Wettbewerbsanreize oder im Hinblick auf Verbesserungsanreizen aus eigenem Antrieb erzielen. Insbesondere kann dadurch auch die Eigenmotivation einer sportausübenden, das angegebene System nutzenden Person 1 gesteigert werden.

Das computerimplementierte Evaluierungsmittel 21 kann entsprechend einer zweckmäßigen Ausführungsform auch dazu ausgebildet sein, einen Vergleich von Leistungsfähigkeits- oder Geschicklichkeitswerten und/oder von festgestellten Niveaus an Leistungsfähigkeit oder Geschicklichkeit in Bezug auf einzelne, mehrere oder alle einer vordefinierten Vergleichsgruppe angehörenden Personen vorzunehmen. Insbesondere kann vorgesehen sein, dass via das computerimplementierte Evaluierungsmittel 21 eine Gruppe von Personen gebildet ist bzw. definiert werden kann, welche sodann als Community bzw. als sogenannte Vergleichsgruppe geführt ist. Dadurch kann eine Competition (Wettbewerb) ausgeführt bzw. ein Wettkampfeffekt generiert und eine Verbesserung der sportlichen Leistungsfähigkeit der Teilnehmer dieser Personengruppe erzielt werden.

Wie vorhergehend erläutert, kann das der sportausübenden Person 1 zugeordnete, tragbare mobile Endgerät 6 eine Mehrfachfunktion erfüllen. Insbesondere kann es einerseits Bestandteil des elektronischen Erfassungssystems 3,3' sein und andererseits auch Bestandteil des computerimplementierten Evaluierungsmittels 21 sein, wie dies auch den Fig. 1 und 3 zu entnehmen ist. Das tragbare mobile Endgerät 6 kann dabei als Wearable-Computer verstanden werden und ist typischerweise als Smartphone 7 ausgeführt. Alternativ oder auch in Kombination zu einem Smartphone 7 kann das tragbare mobile Endgerät 6 auch durch eine Smartwatch, durch Smartglasses (Datenbrille), durch eine persönlichen elektronischen Assistenten, oder dergleichen definiert sein.

Die Ausführungsbeispiele zeigen mögliche Ausführungsvarianten, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Der Ordnung halber sei abschließend darauf hingewiesen, dass zum besseren Verständnis des Aufbaus Elemente teilweise unmaßstäblich und/oder vergrößert und/oder verkleinert dargestellt wurden.

**Bezugszeichenaufstellung**

| | | | |
|---|---|---|---|
| 1 | Person | 24 | Video |
| 2 | Sportschuhe | 25 | Hörbuch |
| 3, 3' | elektronisches Erfassungssystem | 26 | Informationsdokument |
| 4 | Druckerfassungssensoren | 27 | Ausgabevorrichtung |
| 5 | elektronische Auswertungseinheit | 28 | Display |
| 6 | mobiles Endgerät | 29 | Lautsprecher |
| 7 | Smartphone | 30 | Vibrationsgenerator |
| 8 | erste Kommunikationsschnittstelle | 31 | Hostrechner |
| 9 | Internet | 32 | Internetportal |
| 10 | Rechnersystem | 33 | Pausenempfehlung |
| 11 | Lehrmaterialien | 34 | Speichervorrichtung (lokal) |
| 12 | Internet-Datenverbindung | 35 | Speichervorrichtung (fernzugreifbar) |
| 13 | elektrische Leitungsverbindungen | | |
| 14 | Nahbereichs-Kommunikationsschnittstelle | | |
| 15 | Nahbereichs-Kommunikationsschnittstelle | | |
| 16 | Nahbereichs-Kommunikationsstrecke | | |
| 17, 17' | Positionserfassungssystem | | |
| 18 | Temperatursensor | | |
| 19, 19' | gyroskopischer Sensor | | |
| 20 | Leistungsfähigkeits- oder Geschicklichkeitswerte | | |
| 21 | computerimplementiertes Evaluierungsmittel | | |
| 22 | Selektions- oder Vorselektionsschritt | | |
| 23 | Gewichtungs- und/oder Zusammenführungsschritt | | |

## Patentansprüche

1. Verfahren zum Unterstützen einer Person (1) bei der Ausübung von Skisport, umfassend die Schritte:
- Erfassen der derzeitigen sportlichen Leistungsfähigkeits- oder Geschicklichkeitswerte (20) der sportausübenden Person durch ein elektronisches Erfassungssystem (3, 3'), welches elektronische Erfassungssystem (3, 3') ein von der Person (1) tragbares mobiles Endgerät (6), insbesondere ein Smartphone (7), umfasst;
- Evaluieren der vom elektronischen Erfassungssystem (3, 3') bereitgestellten Zustandsinformationen oder Ist-Daten durch ein computerimplementiertes Evaluierungsmittel (21);
- vollautomatisches Feststellen oder halbautomatisches Bestimmen eines derzeitigen Niveaus an Leistungsfähigkeit oder Geschicklichkeit der Person (1) durch das computerimplementierte Evaluierungsmittel (21);
- Selektion oder Vorselektion von Lehrmaterial (11) durch das computerimplementiere Evaluierungsmittel (21) aus einer Vielzahl von für das computerimplementierte Evaluierungsmittel (21) zugreifbaren oder verfügbaren Lehrmaterialien (11), wobei diese Selektion oder Vorselektion durch das computerimplementierte Evaluierungsmittel (21) unter Berücksichtigung oder Einbeziehung des automatisiert oder teilautomatisiert ermittelten Niveaus an Leistungsfähigkeit oder Geschicklichkeit der Person (1) vorgenommen wird;
- Ausgeben von selektiertem Lehrmaterial (11) auf dem von der sportausübenden Person (1) tragbaren mobilen Endgerät (6).

2. Verfahren nach Anspruch 1, **gekennzeichnet durch** automatisierte Selektion oder Vorselektion von beispielsweise videobasiertem Lehrmaterial (11), welches Lehrmaterial (11) zur Verbesserung der derzeitigen sportlichen Leistungsfähigkeits- oder Geschicklichkeitswerte (20) vorgesehen ist.

3. Verfahren nach Anspruch 1 oder 2, **gekennzeichnet durch** eine Anzeige von Videobildern und/oder textbasierten Kommentaren auf einem Display (28) des tragbaren mobilen Endgerätes (6) und/oder durch eine Ausgabe von Sprachinstruktionen mittels einem Lautsprecher (29) des tragbaren mobilen Endgeräts (6).

4. Verfahren nach einem der vorhergehenden Ansprüche, **gekennzeichnet durch** Abrufen von videobasiertem Lehrmaterial (11) via eine erste Daten- oder Kommunikationsschnittstelle (8) des tragbaren mobilen Endgeräts (6), welche erste Daten- oder Kommunikationsschnittstelle (8) zur Fernübertragung und zum Fernempfang von Daten gegenüber zumindest einem Hostrechner (31) oder Internetportal (32) ausgebildet ist.

5. Verfahren nach einem der vorhergehenden Ansprüche, **gekennzeichnet durch** eine darstellerische Überlagerung von Grafiken, Symbolen oder Hinweistexten gegenüber videobasiertem Lehrmaterial (11) in Abhängigkeit von automatisiert festgestellten Ausführungsdefiziten der zu unterstützenden Person (1).

6. Verfahren nach einem der vorhergehenden Ansprüche, **gekennzeichnet durch** computerimplementierte Ermittlung und Ausgabe einer Ermüdungsquantifikation und/oder Pausenempfehlung (33) am tragbaren mobilen Endgerät (6).

7. Verfahren nach Anspruch 6, **gekennzeichnet durch** Ausgabe von visuell, akustisch und/oder taktil wahrnehmbaren Signalen mittels dem Display (28), dem Lautsprecher (29) und/oder dem Vibrationsgenerator (30) des tragbaren mobilen Endgeräts (6).

8. Verfahren nach einem der Ansprüche 6 und 7, **gekennzeichnet durch** Einbeziehung von Parametern ausgewählt aus der Gruppe umfassend Ausübungszeitdauer, Geschwindigkeitsprofil, Beschleunigungsprofil, Kräfteprofil, Sportgeräte-Aufkantwinkel, Skistil, Wetterverhältnisse, Pistenzustände und Pistenschwierigkeitsgrade für die computerimplementierte Ermittlung einer Ermüdungsquantifikation und/oder Pausenempfehlung (33).

9. Verfahren nach einem der vorhergehenden Ansprüche, **gekennzeichnet durch** Hinterlegen von erfassten Leistungsfähigkeits- oder Geschicklichkeitswerten (20) und/oder von festgestellten Niveaus an Leistungsfähigkeit oder Geschicklichkeit in einer lokalen Speichervorrichtung (34) des tragbaren mobilen Endgeräts (6) und/oder in einer externen, fernzugreifbaren Speichervorrichtung (35).

10. Verfahren nach einem der vorhergehenden Ansprüche, **gekennzeichnet durch** Vergleichen von zeitlich zurückliegenden, in einer Speichervorrichtung (34; 35) hinterlegten Leistungsfähigkeits- oder Geschicklichkeitswerten (20) und/oder von festgestellten Niveaus an Leistungsfähigkeit oder Geschicklichkeit mit derzeitigen Leistungs- oder Geschicklichkeitswerten (20) und/oder Niveaus an Leistungsfähigkeit oder Geschicklichkeit mittels dem computerimplementierten Evaluierungsmittel (21).

11. Verfahren nach einem der vorhergehenden Ansprüche, **gekennzeichnet durch** Vergleichen von Leistungsfähigkeits- oder Geschicklichkeitswerten (20) und/oder von festgestellten Niveaus an Leistungsfähigkeit oder Geschicklichkeit in Bezug auf einzelne oder mehrere einer vordefinierten Vergleichsgruppe angehörenden Personen.

12. Verfahren nach einem der vorhergehenden Ansprüche, **gekennzeichnet durch** Auswerten von Drucksignalen von wenigstens einem Druckerfassungssensors (4) mittels wenigstens einer elektronischen Auswertungseinheit (5), welcher wenigstens eine Druckerfassungssensor (4) in oder an einem von der Person (1) tragbaren Sportschuh (2) angeordnet ist.

13. Elektronisches System zum Unterstützen einer Person (1) bei der Ausübung von Skisport, umfassend
- ein elektronisches Erfassungssystem (3, 3') eingerichtet zum Erfassen der derzeitigen sportlichen Leistungsfähigkeits- oder Geschicklichkeitswerte (20) der sportausübenden Person, welches elektronische Erfassungssystem (3, 3') ein von der Person (1) tragbares mobiles Endgerät (6), insbesondere ein Smartphone (7), umfasst;
- ein computerimplementiertes Evaluierungsmittel (21) eingerichtet zum Evaluieren der vom elektronischen Erfassungssystem (3, 3') bereitgestellten Zustandsinformationen oder Ist-Daten;
- wobei das computerimplementierte Evaluierungsmittel (21) weiters eingerichtet ist zum vollautomatischen Feststellen oder halbautomatischen Bestimmen eines derzeitigen Niveaus an Leistungsfähigkeit oder Geschicklichkeit der Person (1);
- und wobei das computerimplementiere Evaluierungsmittel (21) weiters eingerichtet ist zur Selektion oder Vorselektion von Lehrmaterial (11) aus einer Vielzahl von für das computerimplementierte Evaluierungsmittel (21) zugreifbaren oder verfügbaren Lehrmaterialien (11), wobei diese Selektion oder Vorselektion durch das computerimplementierte Evaluierungsmittel (21) unter Berücksichtigung oder Einbeziehung des automatisiert oder teilautomatisiert ermittelten Niveaus an Leistungsfähigkeit oder Geschicklichkeit der Person (1) vorgenommen wird;
- und wobei das tragbare mobile Endgerät (6) der sportausübenden Person (1) zum Ausgeben von selektiertem Lehrmaterial (11) eingerichtet ist.

14. Elektronisches System nach Anspruch 13, **dadurch gekennzeichnet, dass** es wenigstens einen Druckerfassungssensor (4) in oder an einem von der Person (1) tragbaren Sportschuh (2) und wenigstens eine elektronische Auswertungseinheit (5) für die Drucksignale des wenigstens einen Druckerfassungssensors (4) umfasst.

15. Elektronisches System nach Anspruch 13 oder 14 mit Mitteln, die dazu eingerichtet sind, die Schritte des Verfahrens nach einem oder mehreren der Ansprüche 1 bis 12 auszuführen.
